# EUROPEAN PATENT SPECIFICATION

(11) **EP 1 114 060 B1**
(45) Date of publication and mention of the grant of the patent: **11.06.2003**
(21) Application number: 99948817.4
(22) Date of filing: 15.09.1999
(51) Int. Cl.: C07K 1/12, C07K 1/18, C07K 7/08, C07K 14/47, C07K 14/79, A61K 38/10, A61K 38/01, C07K 14/315, C07K 1/22

(54) **PROCESS FOR PRODUCING PEPTIDES FROM BIOLOGICAL FLUIDS AND PEPTIDES OBTAINABLE BY SAID PROCESS**
VERFAHREN ZUR HERSTELLUNG VON PEPTIDEN AUS BIOLOGISCHEN FLÜSSIGKEITEN UND NACH DIESEM VERFAHREN HERSTELLBARE PEPTIDE
PROCEDE DE PRODUCTION DE PEPTIDES A PARTIR DE FLUIDES BIOLOGIQUES ET PEPTIDES PRODUITS SELON LEDIT PROCEDE

(30) Priority: 15.09.1998 EP 98203107; 08.06.1999 EP 99201815
(43) Date of publication of application: 11.07.2001
(73) Proprietor: NIZO food research, 6718 ZB Ede (NL)
(72) Inventor: VISSER, Servaas, NL-6713 ML Ede (NL); RECIO, Isidra, NL-6718 ZB Ede (NL)
(74) Representative: Huygens, Arthur Victor
(86) International application number: EP9907002
(87) International publication number: WO00015655

(56) References cited:
- EP-A- 0 519 726
- EP-A- 0 556 083
- WO-A-95/25437
- WO-A-97/16460
- DATABASE WPI Section Ch, Derwent Publications Ltd., London, GB; Class D13, AN 1973-19518U XP002128234 & JP 48 010543 B (AZUMA H)
- DATABASE WPI Section Ch, Week 9201 Derwent Publications Ltd., London, GB; Class B04, AN 92-002669 XP002097190 & JP 03 255095 A (KANEBO LTD), 13 November 1991 (1991-11-13)
- DATABASE WPI Section Ch, Week 9621 Derwent Publications Ltd., London, GB; Class B04, AN 96-205535 XP002097191 & JP 08 073499 A (SNOW BRAND MILK PROD CO LTD), 19 March 1996 (1996-03-19)
- Z.-Y QIAN ET AL: "Isolation and characterisation of sheep lactoferrin, an inhibitor of platelet aggregation and comparison with human lactoferrin" BIOCHIMICA AND BIOPHYSICA ATA, vol. 1243, 1995, pages 25-32, XP002098054
- DATABASE WPI Section Ch, Week 199722 Derwent Publications Ltd., London, GB; Class B04, AN 1997-241659 XP002128150 & JP 09 077681 A (NIPPON SEISHI KK), 25 March 1997 (1997-03-25)
- LEONIL J ET AL: "STUDY OF TRYPTIC HYDROLYSIS OF BOVINE B-CASEIN WITH THE AIM OF PREPARING PEPTIDES WITH BIOLOGICAL ACTIVITIES IN MEMBRANE REACTORS" COLLOQUES INSERM, vol. 174, 1989, pages 65-68, XP002030853

### SEQUENCE LISTING

<110> NIZO food research
<120> Process for producing peptides from biological fluids and peptides obtainable by said process
<140> PCT/EP99/
   <141> 1999-09-15
<150> EP 98203107.2
   <151> 1998-09-15
<150> EP 99201815.0
   <151> 1999-06-08
<160> 8
<170> PatentIn Ver. 2.2
<210> 1
   <211> 16
   <212> PRT
   <213> Artificial Sequence
<220>
   <223> Description of Artificial Sequence: internal fragment from bovine alpha-s2 casein
<400> 1
   Val Tyr Gln His Gln Lys Ala Met Lys Pro Trp Ile Gln Pro Lys Thr
   1 5 10 15
<210> 2
   <211> 21
   <212> PRT
   <213> Artificial Sequence
<220>
   <223> Description of Artificial Sequence: internal fragment from bovine alpha-s2 casein
<400> 2
   Val Tyr Gln His Gln Lys Ala Met Lys Pro Trp Ile Gln Pro Lys Thr
   1 5 10 15
   Lys Val Ile Pro Tyr
   20
<210> 3
   <211> 24
   <212> PRT
   <213> Artificial Sequence
<220>
   <223> Description of Artificial Sequence: internal fragment from bovine alpha-s2 casein
<400> 3
   Val Tyr Gln His Gln Lys Ala Met Lys Pro Trp Ile Gln Pro Lys Thr
   1 5 10 15
   Lys Val Ile Pro Tyr Val Arg Tyr
   20
<210> 4
   <211> 25
   <212> PRT
   <213> Artificial Sequence
<220>
   fragment from bovine alpha-s2 casein
<400> 4
   Val Tyr Gln His Gln Lys Ala Met Lys Pro Trp Ile Gln Pro Lys Thr
   1 5 10 15
   Lys Val Ile Pro Tyr Val Arg Tyr Leu
   20 25
<210> 5
   <211> 29
   <212> PRT
   <213> Artificial Sequence
<220>
   <223> Description of Artificial Sequence: internal fragment from goat whey
<220>
   <221> DISULFID
   <222> (6) .. (23)
<400> 5
   Pro Glu Trp Ser Lys Cys Tyr Gln Trp Gln Arg Arg Met Arg Lys Leu
    1               5                  10                  15
   Gly Ala Pro Ser Ile Thr Cys Ile Arg Arg Thr Ser Ala
               20                  25
<210> 6
   <211> 29
   <212> PRT
   <213> Artificial Sequence
<220>
   <223> Description of Artificial Sequence: internal fragment from goat whey, chemically modified
<400> 6
   Pro Glu Trp Ser Lys Cys Tyr Gln Trp Gln Arg Arg Met Arg Lys Leu
    1               5                  10                  15
   Gly Ala Pro Ser Ile Thr Cys Ile Arg Arg Thr Ser Ala
               20                  25
<210> 7
   <211> 16
   <212> PRT
   <213> Artificial Sequence
<220>
   <223> Description of Artificial Sequence: internal fragment from sheep whey
<400> 7
   Thr Gln Arg Lys Thr Arg Asn Gly Phe Arg Val Pro Leu Ala Arg Glu
    1               5                  10                  15
<210> 8
   <211> 8
   <212> PRT
   <213> Artificial Sequence
<220>
   <223> Description of Artificial Sequence: N-terminal fragment from bovine whey
<400> 8
   Ala Pro Arg Lys Asn Val Arg Trp
    1               5

## Description

### Field of the Invention

The present invention relates to a process for the production of peptides of interest from proteins which occur in biological fluids. Said process involves the separation of proteins from fluids such as milk, whey or blood, by some form of chromatography, followed by further steps comprising fragmenting these proteins *and* selecting the peptides of interest. The invention also relates to peptides which are obtainable by said process.

### Background of the Invention

In recent years, it has become widely recognised that many organisms, from prokaryotes to humans, use peptides as part of their host defense system. Among these, cationic peptides form an important class showing a variety of antimicrobial properties. Different peptides may have antibacterial, antiendotoxic, antibiotic-potentiating or antifungal properties, and so they are being developed for use as a novel class of antimicrobial agents and as the basis for making transgenic disease-resistant plants and animals. For a review, see, e.g., R.E.W. Hancock and R. Lehrer, *Tibtech* (1998) 16:82-88.

W. Lee Maloy and U. Prasad Kari, *Biopolymers* (1995) 37:105-122 report that the structures of these peptides from insects, horseshoe crabs, frogs, and mammals are known to have in common features of a net cationic charge due to the presence of multiple Arg and Lys residues and in most cases the ability to form amphipathic structures. Despite very diverse peptide sequences and structures, most host defense peptides appear to act by a direct lysis of the cell membrane of a pathogen. Their basic structure facilitates their interaction with the cell membrane, and their amphipathic character allows them to be incorporated into the membrane, ultimately disrupting its structure. These authors divide the host defense peptides in two structural classes, linear peptides and cyclic peptides with one to three disulfide bonds. A further tentative classification of antimicrobial peptides into four distinct groups is given by D. Destoumieux *et al., J. Biol. Chem. (1997)* 45:28398-28406. These groups are based on amino acid sequences, secondary structures, and functional similarities: (i) linear basic peptides forming amphipathic α-helices, (ii) peptides with one to six intramolecular disulfide bridges, (iii) proline-rich peptides, and (iv) glycine-rich antimicrobial peptides or polypeptides (9-30 kDa). It is referred to that the mode of action, the broad activity, the molecular diversity, and the noncytotoxicity of all these circulating antimicrobial peptides make them very attractive as therapeutic agents for pharmaceutical or agricultural applications.

W.E. Robinson *et al, J. Leukoc. Biol.* (1998) 63:94-100, report anti-HIV-1 activity of indolicidin, an antimicrobial tridecapeptide amide isolated from the cytoplasmic granules of bovine neutrophils. Similarly, D. Winder *et al., Biochem. Biophys. Res. Commun.* (1998) 242:608-612, disclose that the expression of genes encoding the (cationic) antimicrobial peptides cecropin and mellitin has an antitumour effect in human cells.

K. Natarajan and J.A. Cowan, *Chemistry and Biology* (1998) 5:147-154 postulate on the basis of the solution structure of a synthetic lytic peptide corresponding to the amino terminus of human perforin, that the strong electrostatic interaction between the cationic region of said peptide and the lipid headgroups probably weakens the membrane, facilitating insertion of the relatively neutral/hydrophobic stretch of said peptide, and is representative of the lytic pathway. It is stated that this structural model is probably relevant to understanding the mechanisms of other cationic antimicrobial peptides.

T. Wieprecht *et al*., *Biochemistry* (1997) 36:12869-12880 report on the influence of the angle subtended by the positively charged helix face on the membrane activity of amphipathic, antibacterial peptides. An amphipathic, mostly α-helical conformation and a positive net charge have been recognised as major structural motifs which determine the membrane disturbing activity. Enhancement of the total cationic peptide charge often results in a higher antibacterial activity.

Lactoferrin is an iron-binding protein that is present in the whey fraction of milk. This protein is a main antimicrobial component of milk and is thought to contribute to the protection of infants from infectious diseases. A large number of studies has demonstrated its strong bacteriostatic effect and sometimes its bactericidal effect and its antiviral activity. Lactoferrin may relieve some of the effects of inflammation and has a role in regulating various components of the immune system. For a review, see, e.g., L. Sanchez *et al., Arch. Dis. Child.* 67:657-661.

Some bioactive peptides from lactoferrin have been reported. For example, in the early 90s a tetrapeptide of human lactoferrin was described as an inhibitor of platelet aggregation, of fibrinogen binding to ADP-activated platelets and of serotonin release: it was antithrombotic in rats, guinea-pigs and in dogs. See E. Mazoyer *et al., Eur. J. Biochem.* (1990) 194:43-49; L. Drouet *et al., Nouv. Rev. Fr. Hematol.* (1990) 32:59-62; G. Wu *et al., Haemostasis* (1992) 22:1-6.

Some peptides of human or bovine lactoferrin possess antibacterial properties. It was found that a pepsin-generated hydrolysate of lactoferrin displayed bactericidal properties that were more potent than those of undigested lactoferrin. Also, a potent antibacterial peptide fragment from the N-terminal part of lactoferrin, named lactoferricin, was isolated from that hydrolysate. See, e.g., W. Bellamy *et al., J. Appl. Bacteriol.* (1992) 73:472-479 and *Biochim. Biophys. Acta* (1992) 1121:130-136; M. Tomita *et al., J. Dairy Sci.* (1991) 74:4137-4142; K. Yamauchi *et al., Infect. and Immun.* (1993) 61:719-728; and E.M. Jones et *al., J. Appl. Bacteriol.* (1994) 77:208-214.

Y.-C. Yoo *et al., Biochem. Biophys. Res. Commun.* (1997) 297:624-628 report on the activity of bovine lactoferricin to induce apoptosis in THP-1 human monocytic leukemic cells.

In JP 08 073499 A (publ. March 1996) peptides are disclosed which are derived from human lactoferrin and which are said to be useful as immunoactivators, especially for preventing infection by cytomegalovirus.

Z.-Y. Qian *et al.*, *Biochim. Biophys. Acta* (1995) 1243:25-32 disclose the isolation and characterisation of pepsin hydrolysates of sheep and human lactoferrin and their inhibiting effect on platelet aggregation. It was *inter alia* found that a positive charge and a certain conformation of peptides are very important for the binding of lactoferrin to their receptors at the surface of platelets.

It has been documented that casein derived phosphopeptides which generally are highly negatively charged may play an important role as inhibitors of intra-intestinal calcium nucleation and precipitation. See D.G. Schmidt *et al., Neth. Milk Dairy J.* (1987) 41:121-136, and R. Sato *et al., Biochim. Biophys. Acta* (1991) 1077:413-415.

WO 87/07615 and US 5,015,628 disclose a particular role of casein phosphopeptides in the prevention of dental caries, whereas P. Schupbach *et al., J. Dent. Res.* (1996) 75:1779-1788, report a similar potency of the likewise negatively charged caseinomacropeptide.

P.W.J.R. Caessens *et al., J. Agric. Food Chem.* (1999) 47:1856-1862, report that an amphipathic peptide from bovine β-casein carrying a highly negatively charged N-terminal sequence, contributes to the emulsion-stabilizing properties of a biological system.

The prior art referred to above and the references cited therein show abundantly the interest in the functional properties including the structure-activity relationship of naturally occurring peptides or peptide fragments, in particular in relation to the immune responses of humans and animals.

A variety of methods for the preparation and isolation of lactoferrin and its hydrolysates have been described in the prior art. See, for example, WO 89/04608, WO 93/02098, WO 93/13676, EP-A-0253395, EP-A-0556083, FR 2613725 and NL 8601814, wherein *inter alia* techniques are applied for the separation of charged molecules which include cation-exchange means.

EP-A-0519726 discloses a process for large-scale production of an antimicrobial peptide in high purity which comprises the steps of first isolating the protein containing the amino acid sequence of the peptide of interest, contacting a mixture of peptides containing the antimicrobial peptide obtainable by hydrolysing isolated bovine lactoferrin or bovine milk protein containing lactoferrin, with a hydrophobic chromatographic medium or cation-exchange chromatographic medium to adsorb the antimicrobial peptide, washing the medium to elute peptides other than the antimicrobial peptide, desorbing the antimicrobial peptide in solution from the washed medium, and, optionally, desalting the desorbed antimicrobial peptide solution, whereby the antimicrobial peptide of at least 90% by weight purity is obtained. This process is rather complex and therefore expensive, and one can really wonder if for many applications an antibacterial product based on lactoferrin having a somewhat lower specific activity as compared to lactoferrin but produced in a relatively simple and therefore cheaper way, would not suffice.

Similar methods of preparing active peptides by cleaving a starting protein such as casein in milk enzymatically into smaller fragments, followed by a chromatographic purification step are disclosed in WO 97/16460 and JP 03 255095 A. These methods are distinct from the method of the present invention and result in different peptide fragments. WO 97/16460 disclose casein fragments having growth promoting activity, whereas JP 03 255095 A disclose casein peptides for treating thrombosis, as inhibitors of platelet aggregation caused by adenosine-5'-diphosphate and collagen.

WO 95/25437 discloses vegetable protein hydrolysates with improved colour quality which are obtained by a) extracting protein-containing vegetable flours at a pH beyond the isoelectrical range of the protein, possibly in the presence of adsorbents and b) hydrolysing the resulting protein hydrolysate in the presence of adsorbents with alkalis, acids and/or enzymatically in a manner known per se.

JP 48 010543 B (Derwent 1973-19518U) discloses the manufacture of edible protein with proteolytic enzyme by hydrolysing animal protein, exemplified by mackerel meat, in the presence of adsorbents (acid clay, active charcoal, bentonite, etc) and/or ion-exchange resin, resulting in a hydrolysate without bitterness or malodour. The resulting hydrolysate was heated to inactivate the enzyme and pasteurised, and the solid and fats and oils were separated. The solution which had no bitter taste was further concentrated under reduced pressure to give a powder containing 89% protein.

J. Leonil *et al., Colloques INSERM*(1989) 174:65-68 disclose the tryptic digestion of the milk protein β-casein in an enzymatic membrane reactor, resulting in biologically active peptides with different release rates of fragments in the time.

JP 09 077681 A (Derwent 1997-241659) discloses an anti-HIV agent containing the peptide nisin from *Streptococcus lactis* as active component.

In view of the increased interest in peptides with useful and promising properties, there is therefore an increasing need for an efficient way to produce such peptides. The present invention aims to provide such a method which is both relatively simple and cheap.

### Summary of the Invention

In accordance with an aspect of the present invention, there is provided a process for the production of at least one peptide of interest from a biological fluid which comprises the steps of:
a) contacting said biological fluid comprising one or more proteins which contain the amino acid sequence of said peptide of interest with a chromatographic medium to adsorb said protein containing said sequence,
b) subjecting said adsorbed material to hydrolysis to fragment said protein and produce said peptide which remains substantially adsorbed,
c) washing the medium to remove unbound material,
d) desorbing said remained substantially adsorbed peptide from said chromatographic medium, and, optionally,
e) further purifying said desorbed peptide of interest.

Preferably, the medium is also washed before the hydrolysis step to remove unbound material. The chromatographic medium preferably is an ion-exchanger or some affinity column material, and most preferably a chromatographic membrane having similar properties. The hydrolysis preferably is carried out enzymatically. Suitable enzymes include, e.g., pepsin, chymosin, trypsin, plasmin, chymotrypsin, subtilisin and thermolysin. Preferred biological fluids include milk, whey and other milk products, blood, blood serum, egg white, culture cells, extracts from culture cells, and plant cells.

In another aspect of the invention various peptides are provided which are obtainable by the present process. A number of said peptides is novel. In a preferred embodiment said peptides are in substantially pure form. In another preferred embodiment said peptides are biologically active.

In still another aspect of the invention said peptides are suitable for use as pharmaceutical compositions (including the pharmaceutically or sitologically acceptable salts) for various therapeutic and diagnostic purposes, for example based on antimicrobial, antiviral or antitumour activity.

These and other embodiments and advantages of the invention will become apparent to those skilled in the art upon review of the following detailed description and claims.

### Brief Description of the Drawings

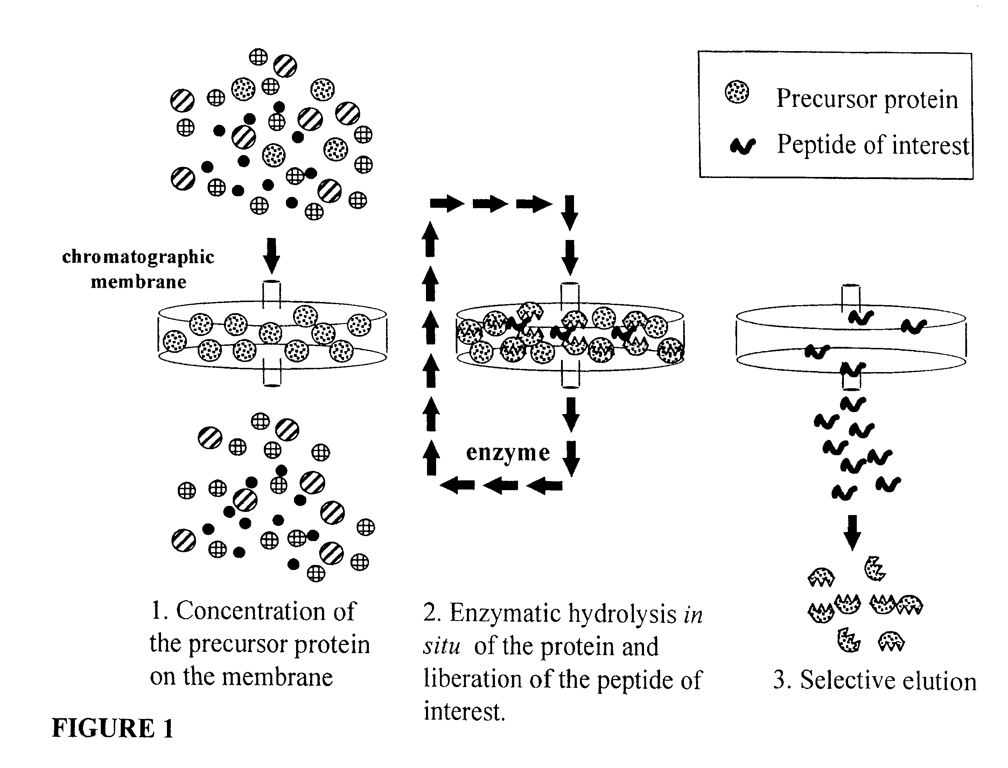

Figure 1 is a schematic and illustrative representation of a preferred embodiment of the process according to the present invention.
Figure 2 is an RP-HPLC chromatogram of the active product obtained after hydrolysis of bovine α_{S2}-casein bound to a cation-exchange membrane (starting from a solution of α_{S2}-casein).
Figure 3 is an RP-HPLC chromatogram of the active product obtained after hydrolysis of goat whey protein bound to a cation-exchange membrane (starting from microfiltered goat whey).
Figure 4 is an RP-HPLC chromatogram of the active product obtained after hydrolysis of sheep whey protein bound to a cation-exchange membrane (starting from microfiltered sheep whey).
Figure 5 is an RP-HPLC chromatogram of the active product obtained after hydrolysis of bovine whey protein bound to a cation-exchange membrane (starting from microfiltered bovine whey).
Figure 6 is an RP-HPLC chromatogram of the active product obtained after hydrolysis of bovine lactoferrin bound to a cation-exchange membrane (starting from a solution of bovine lactoferrin).
Figure 7 is an RP-HPLC chromatogram of (a) the cell-free supernatant containing nisin A precursor, (b) the active product obtained after hydrolysis of nisin A precursor bound to a cation-exchange membrane.
Figure 8 is an RP-HPLC chromatogram of the fraction containing negatively charged peptides retained after hydrolysis of bovine β-casein bound to an anion-exchange membrane (starting from a solution of bovine β-casein).
Figure 9 is an RP-HPLC chromatogram of the fraction containing positively charged peptides retained after hydrolysis of egg white protein bound to a cation-exchange membrane (starting from a solution of chicken egg white).

In most Figures the observed masses of identified peptides are indicated.

### Detailed Description of the Invention

Asymmetric clustering of basic residues has been observed in various peptides that show an affinity for biological membranes. These cationic peptides have been shown, besides other activities, to kill sensitive microorganisms by inducing an increase in cell membrane permeability. The isolation of these peptides from protein hydrolysates is generally laborious, because it implies first the isolation of the precursor protein and then the purification of the cationic peptides from the enzymatic protein hydrolysate. The same applies for the isolation and purification of anionic or hydrophobic peptides having particular properties of interest.

As used herein, the term "peptide" generally refers to a protein fragment without being bound to a certain size, thus including "oligopeptide" and "polypeptide". The term "peptide of interest", as used herein, refers to a peptide having any kind of activity, and most preferably a biological activity. The term "medium", as used herein in connection with chromatographic techniques, generally refers to a resin, gel or membrane, or other conventional chromatographic means. The use of membranes is generally preferred.

In accordance with the present invention it has surprisingly been found, after extensive research and experimentation, that a peptide of interest or a fraction enriched in such peptides of interest can be directly and efficiently obtained from a biological fluid in which proteins are contained which comprise such peptide(s) of interest, by a process comprising the steps of (i) contacting said biological fluid with a chromatographic medium (ii) hydrolysing *in situ* the selectively bound protein, i.e. hydrolysis on said chromatographic medium, without the need for intermediate isolation of the precursor protein(s) of interest, (iii) washing out the unbound peptides, (iv) selectively eluting the peptide or peptides of interest from said chromatographic medium, and, optionally (v) further purifying the resulting peptide or peptides.

The starting material used in the present process is any protein or mixture of proteins containing relevant domains within their sequences. The process is particularly suitable when such proteins or mixtures of proteins are obtained from biological fluids such as milk, whey or other milk fractions, blood or blood serum, egg white, cell cultures or cell culture extracts, or solutions of plant proteins. In a preferred embodiment of the invention the cell cultures or cell culture extracts are obtained from wild-type or genetically modified host organisms (e.g. bacteria, yeasts, fungi, cell lines, or plant cells) and comprise one or more precursor forms of the peptide of interest. However, it will be understood that any fluid comprising proteins which contain a peptide sequence of interest may be used. For the purpose of the present invention, such fluids are also comprised within the term "biological fluid".

If, for instance, the isolation of charged peptides is desired, the starting material is dissolved or dispersed in water or buffer solution, preferably low ionic strength buffer at the appropriate pH to bind the protein of interest to the ion-exchanger. Usually, no purification step is required or needed prior to contacting the solution or dispersion with the ion-exchanger. Any insoluble material, if present, can be removed in a usual manner, such as by microfiltration, ultrafiltration or centrifugation.

Ion-exchange procedures have been described for the isolation of protein/peptide components from fluids. For instance, lactoferrin and lactoperoxidase from cheese whey using bead-based ion-exchange. See the references referred to above.

Either a strong or a weak cation or anion-exchange medium (resin, gel or membrane) can be used for the concentration of the protein of interest employing continuous-flow or batchwise chromatographic techniques. A variety of groups can be chosen for use in cation-exchangers, such as carboxymethyl, methyl sulphonate, sulphopropyl or sulphonic acid. Similarly, for use in anion-exchangers, functional groups such as diethylaminoethyl or quaternary ammonium can be applied. The matrix is usually based on inorganic compounds, synthetic resins, polysaccharides, etc., a strong ion-exchange membrane being preferred. In such a membrane, functional groups are covalently attached to the inner surface of large pore size, non-compressible and chemically very stable microporous cellulose membrane (e.g. Sartorius).

The bound protein is then hydrolysed, preferably by enzymatic hydrolysis. However, acid or alkaline hydrolysis, optionally in combination with enzymatic hydrolysis can also be employed, if desired. The hydrolysis is carried out *in situ*, i.e. without isolating the selectively bound protein. The medium is preferably washed with water and/or an appropriate buffer solution prior to hydrolysis in order to substantially remove unbound materials.

In a preferred embodiment of the invention the selectively bound protein is enzymatically digested with pepsin. However, any enzyme (each at or near its optimum pH) which is capable of cleaving the protein of interest bound to the chromatographic medium to generate peptides of interest may be used, proteases or peptidases being preferred. Suitable enzymes include, for example, chymosin (pH approx. 3.0), trypsin, plasmin, chymotrypsin, subtilisin, and thermolysin (all at neutral pH).

If, for instance, the isolation of cationic peptides is desired, the washing out of the cation-exchanger and preferably the cation-exchange membrane to remove other protein fragments, i.e. mainly non-cationic peptides, is usually commenced immediately after hydrolysis of the protein of interest. This step can be suitably performed with water and/or a buffer solution, preferably low ionic strength buffer, at low or neutral pH, which pH is preferably about one pH unit lower than the pl of the bound cationic peptide or peptides. For instance, the washing out of these fragments can be performed with water or 10 mM sodium phosphate buffer, or alternatively a volatile buffer, e.g. 10 mM ammonium bicarbonate buffer. If desired, this by-product fraction is dried by conventional means, e.g. freeze-drying or spray-drying, and may be further employed. The cationic peptides bound to the membrane are then eluted in a usual way employing one or more appropriate buffer solutions, preferably volatile buffers, e.g. ammonium solutions of different concentrations thus avoiding a desalting step. If necessary, high ionic strength buffers, e.g. 2 M sodium chloride in phosphate buffer can be employed.

The elution step is suitably followed by further purification of the resulting peptide fraction in a manner known per se. For example, if necessary, desalting of the solution containing the peptide or peptides is suitably carried out by techniques such as ultrafiltration, dialysis, electrodialysis with membranes of suitable cut-off, gel permeation chromatography, hydrophobic interaction chromatography, or high performance liquid chromatography. If desired, this latter technique can be employed for further purification of the peptide or peptides.

The peptides obtained by the method of the present invention can be modified, if desired, by conventional techniques. For example, peptides containing cysteine residues frequently have disulfide bridges between these residues. These peptides can be modified by converting the disulfide bridge into the corresponding thiol groups, for example by chemical reduction. The biological activity of the resulting peptides with thiol groups is usually unaffected.

In a typical example, the process according to the present invention was successfully applied to the small scale production of antibacterial peptides derived from lactoferrin (LF), using cheese whey as starting material. Cheese whey from different species (cow, goat or sheep), or a mixture thereof, was employed and this resulted in a fraction of peptides with much higher activity (at least 12-fold more potent) than the undigested LFs. However, it will be clear to those skilled in the art that the present method can be successfully used with any proteins containing peptide fragments of interest, which proteins may or may not be present in complex mixtures in biological fluids.

The chromatography step in the method according to the present invention is preferably carried out using chromatographic membranes, but also other chromatographic techniques such as hydrophobic interaction or affinity chromatography can be employed, either in one or more columns or batchwise. Membrane-based processes are generally preferred since they are more rapid by allowing a higher flow rate (approx. twenty-fold) as compared with bead-based systems. In addition, these membrane-based processes can be easily up-scaled to gram or even kilogram quantities.

The process according to the present invention is relatively simple and therefore generally economically and technically more attractive than the prior art methods. It provides in high yields peptides with a selected activity of interest without the need of intermediate purification of the precursor protein. Besides, by using the method according to the invention which clearly distinguishes from the *prior* art methods a number of novel peptides is obtained. In a preferred embodiment of the invention, the process is used for the production of lactoferricin and derivatives thereof from milk or whey from an appropriate source (human or animal, such as bovine, goat, sheep, horse), having desired properties. Also casein, e.g. purified bovine β- or αₛ₂-casein is a suitable and preferred source for the production of peptides having various kinds of functional properties. Other suitable starting materials are, for example, blood, blood serum, egg white, culture cells and extracts thereof, and plant cells.

In a further aspect of the present invention, there are provided peptides, preferably in a substantially pure form, which are obtainable by the invented process, a number of which are novel. Biologically active peptides are the preferred choice. Biological activities of interest which make these peptides particularly useful include antimicrobial, antiviral, antitumour, antiinflammatory and antithrombotic activity. Evidently, the biological activity *inter alia* depends on the nature of the starting biological fluid and the proteins contained therein. A preferred embodiment of the present invention is the preparation of nisin in any form, for example nisin A or nisin Z, or a mixture thereof. A suitable starting material is, e.g., a precursor form as exemplified in Example 6.

Alternatively, these peptides including the novel peptides can be produced by other methods known in the art, such as by chemical synthesis or microbiologically or by plants.

The invention further provides a peptide of interest, obtainable by the method of the present invention, as described hereinbefore, having an amino acid sequence selected from the following sequences (1) - (8), or derivatives thereof having a primary amide at the carboxy end thereof, which derivatives do not interfere with or abolish any biological properties of the peptide:

Although the process according to the present invention has been predominantly disclosed for the production of cationic peptides, it is also suitable for the production of peptides which are differently charged, or of hydrophobic peptides and peptides that otherwise can be specifically bound to a chromatographic medium based on particular properties.

The present invention also discloses the use of a peptide having an amino acid sequence selected from the sequences (1) - (8), as defined above, or derivatives thereof having a primary amide at the carboxy end thereof, which derivatives do not interfere with or abolish any biological properties of the peptide, for the preparation of pharmaceutical compositions, preferably with antimicrobial and/or antiviral and/or antitumour activity.

The invention will be further illustrated by the following methods and examples which are not to be construed as limiting the invention in any respect.

### Materials and methods

### Analytical Procedures

### 1. Reversed-phase high performance liquid chromatography (RP-HPLC)

Analytical RP-HPLC was carried out using two M 6000A pumps in combination with a high-sensitivity accessory block (Waters Ass.), an ISS-100 injector (Perkin-Elmer), a Waters Model 680 gradient controller and a Kratos 783 detector (Kratos Analytical). A 250 mm x 4.6 mm Widepore C₁₈ column (Bio-Rad Laboratories) was used with a C₁₈ cartridge (Bio-Rad) as guard column. The equipment was linked to a data acquisition and processing system (Turbochrom, Perkin-Elmer). Solvent A was a mixture of acetonitrile-water-trifluoroacetic acid (TFA) (100:900:1, v/v/v) and solvent B contained the same components (900:100:0.8, v/v/v).

Mostly, peptides were eluted using a linear gradient of solvent B in A from 0% to 50% over 70 min at a flow rate of 0.8 ml/min. Fractions of nisin or nisin precursor were eluted with a linear gradient of solvent B in A going from 10% to 25% in 10 min followed by a 50-min linear gradient from 25 to 30% of solvent B in solvent A at a flow rate of 0.8 ml/min. Fractions from β-casein were eluted in the following linear gradient steps: from 10 to 16% of solvent B in A in 2 min followed by 2 min isocratic elution; to 26% B in 8 min; to 35% B in 4 min, and then to 38% B in 42 min. The absorbance of the eluent was monitored at 220 nm. Sample concentrations were approx. 1 mg/ml and injection volumes 50 µl.

### 2. Mass spectrometry (MS) and tandem mass spectrometry (MS/MS)

The mass of each purified peptide was assessed by mass spectrometry using a Quattro II triple quadrupole instrument (Micromass). Samples were dissolved in 50% acetonitrile, 0.3% formic acid and analysed by infusion with a syringe pump type 22 (Harvard Apparatus, South Natick, MA, USA) of the sample solution in 50% acetonitrile at 4 µl/min through the electrospray interface. Nitrogen was used as a nebulizing and drying gas. The capillary was held at 3.9 kV; the cone voltage was 30 V. MS/MS was achieved using 5x10⁻³ mbar of argon in the second quadrupole. Automated peak recognition and daughter ion scanning were performed using the Masslynx software version 2.2 (Micromass) on a Windows NT workstation.

### 3. N-terminal sequence analysis

The N-terminal part of the purified peptides was identified by sequence analysis with a gasphase sequenator (Model 470A, Applied Biosystems Inc.) using 25% TFA in water as conversion agent. The resulting phenythiohydantoin (PTH) amino acids were analysed on-line by RP-HPLC with a PTH analyser (Model 120A, Applied Biosystems Inc.) using a PTH C-18 column (2.1 mm x 220 mm) (Applied Biosystems Inc.).

### 4. Assay for antibacterial activity

Antibacterial activities of the peptide mixtures and/or isolated peptides were determined by a plate diffusion assay using *Micrococcus flavus* DSM 1790 as indicator organism. *M. flavus* was grown in MF broth (0.1% sucrose, 1% peptone, 0.3% meat extract, 0.2% NaCI, 0.15% yeast extract pH 7.0). Peptide mixtures were tested at concentrations from 0.2 mg/ml to 50 mg/ml in bidistilled water.

A microtiter method was used to determine the minimal inhibitory concentrations (MICs) of the peptides in liquid media against various microorganisms, viz. *E. coli* (2 different strains), *Listeria innocua, Bacillus cereus, Micrococcus flavus, and Streptococcus thermophilus.*

### Example 1

### Preparation of a peptide mixture with antibacterial activity from α_{S2}-casein

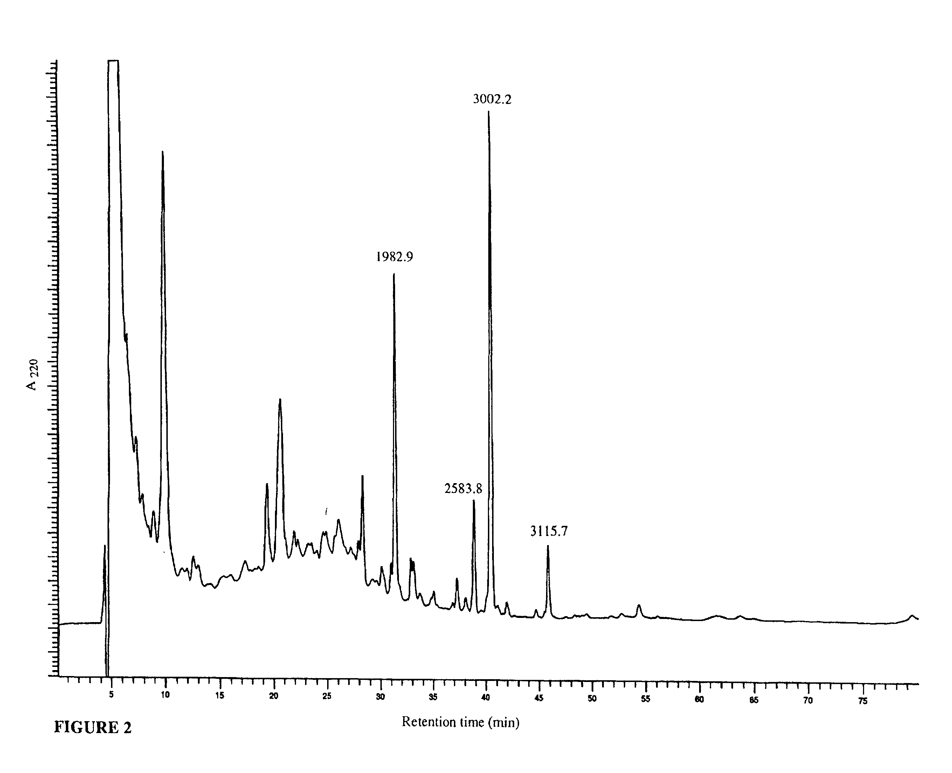

Bovine α_{S2}-casein isolated from milk was dissolved in bidistilled water at a concentration of 0.1 mg/ml and the pH was adjusted to 3.0 by addition of 1 M HCI. Prior to use, the cation-exchange membrane (Sartorius Sartobind S MA 100; Sartorious GmbH, Germany) was pre-equilibrated with acidulate bidistilled water (pH 3.0). The process was monitored by a UV detector with 2-mm lightpath flow cuvette (model EM-1 Econo UV Monitor, Bio-Rad). 1000 ml of the α_{S2}-casein solution was pumped through the cation-exchange membrane at room temperature and at a flow rate of 20 ml/min. The cation-exchange membrane was then washed with acidified water (pH 3.0) to remove unbound material. The α_{S2}-casein bound to the membrane was hydrolysed for 6 hours at 37 °C by recycling at 20 ml/min, at reverse flow, 100 ml of an aqueous solution (25 mg/ml, pH 3.0) of porcine pepsin (Sigma Chemical Co.). The cation-exchange membrane was rinsed with acidified water pH 3.0 followed by 10 mM ammonium bicarbonate buffer, adjusted with formic acid (pH 7.0) until baseline level was achieved. Finally, the cationic antibacterial peptides were desorbed from the membrane with 7 M ammonia solution which was freeze-dried to obtain a powdered active product. This active product was monitored by reversed-phase high performance liquid chromatography (RP-HPLC) (Fig. 2), and the peptides were identified by mass spectrometry (MS) and tandem mass spectrometry (MS/MS). The masses and amino acid sequences of major peptides obtained in this product are summarised in Table 1. After extensive hydrolysis (overnight), the ratio among the four peptides was shifted towards the shortest sequence (mass 1982.9).

**Table 1**

| Masses and amino acid sequences of the cationic antibacterial peptides obtained after hydrolysis of α_{S2}-casein on the cationic-exchange membrane | | |
|---|---|---|
| Observed mass | Theoretical mass | Sequence |
| 1982.9 | 1983.4 | VYQHQKAMKPWIQPKT |
| 2583.8 | 2584.1 | VYQHQKAMKPWIQPKTKVIPY |
| 3002.2 | 3002.6 | VYQHQKAMKPWIQPKTKVIPYVRY |
| 3115.7 | 3115.8 | VYQHQKAMKPWIQPKTKVIPYVRYL |

### Example 2

### Preparation of a peptide with antibacterial activity from goat whey

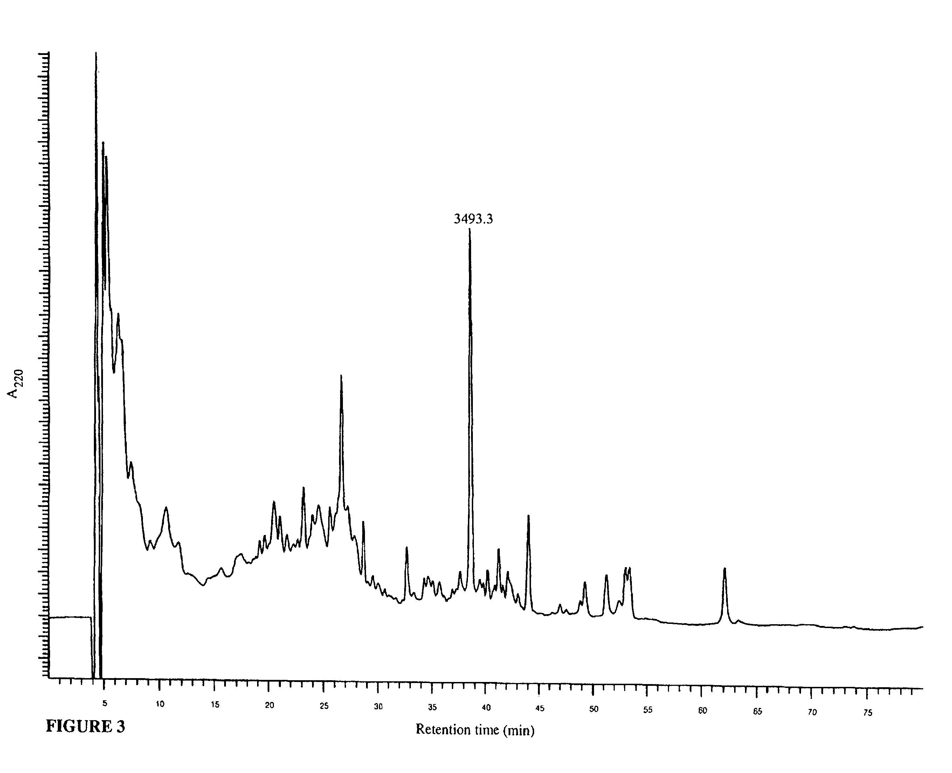

Prior to its use, the cation-exchange membrane described in Example 1 was pre-equilibrated with 10 mM sodium phosphate buffer (pH 7.0). The process was monitored by a UV detector with 2-mm lightpath flow cuvette (model EM-1 Econo UV Monitor, Bio-Rad). 1000 ml of microfiltered goat cheese whey at pH 6.5 (hereafter referred to as "starting material") was pumped through the cation-exchange membrane at room temperature and at a flow rate of 20 ml/min. The cation-exchange membrane was washed with acidified bidistilled water (pH 3.0) or alternatively, bidistilled water or 10 mM phosphate buffer to remove unbound material. The material bound to the cation-exchange membrane was hydrolysed overnight at 37 °C by recycling at 20 ml/min, at reverse flow, 100 ml of an aqueous solution (25 mg/ml, pH 3.0) of porcine pepsin (Sigma Chemical Co.). The cation-exchange membrane was rinsed with acidified water pH 3.0 followed by a 7 M ammonia solution until baseline level was achieved. Finally, the strongly bound peptides were eluted with 2 M NaCl. The 2 M NaCI fraction was desalted using a Sep-Pak® C₁₈ environmental cartridge (Waters Ass.) with a linear 0 to 100% gradient of acetonitrile in water. After acetonitrile evaporation, this fraction was freeze-dried to obtain a powdered active product. This active product was monitored by RP-HPLC (Fig. 3) and MS, and the sequence of the major peptide in this fraction was derived from N-terminal sequencing and MS data. The mass and the sequence of the major peptide in this product are summarised in Table 2. The data indicate that this active peptide is a goat lactoferrin fragment.

**Table 2**

| Mass and amino acid sequence of the cationic antibacterial peptide obtained after hydrolysis on the cationic-exchange membrane using goat cheese whey as starting material | | |
|---|---|---|
| Observed mass | Theoretical mass | Sequence |
| 3493.3 | 3493.9 | PEWSKC*YQWQRRMRKLGAPSITC*IRRTSA |

| | | |
|---|---|---|
| * linked by a disulfide bridge | | |

### Example 3

### Preparation of a peptide with antibacterial activity from sheep whey

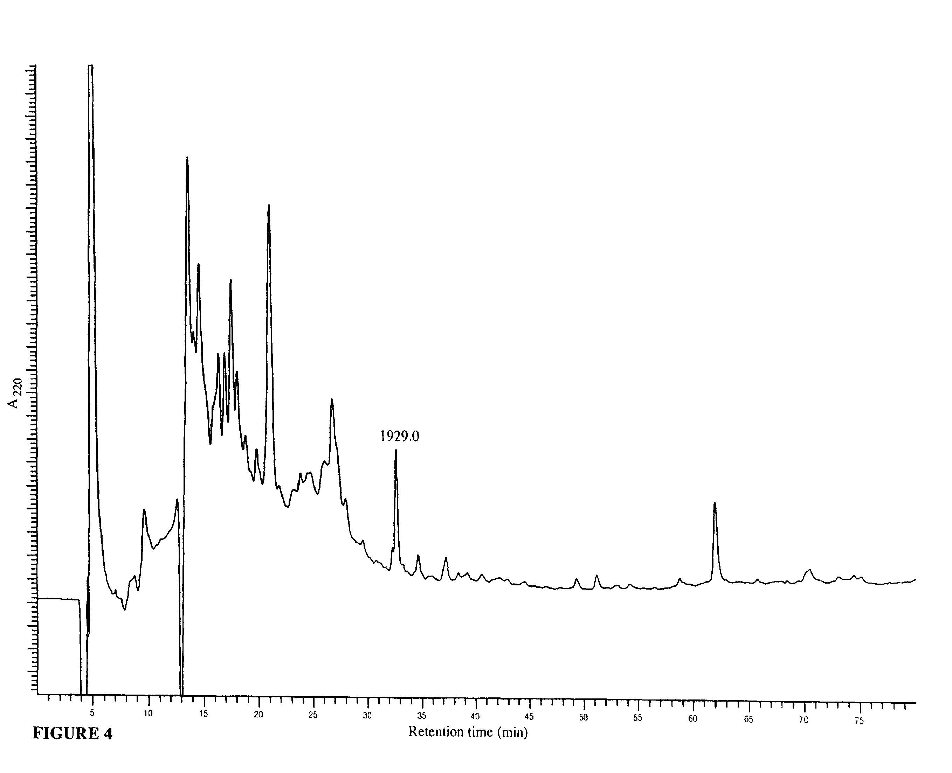

Experimental conditions were as described above for Example 2 with the exception that microfiltered sheep cheese whey (pH 6.5) was used as starting material. The active product obtained was monitored by RP-HPLC (Fig. 4) and MS, and the sequence of the major peptide in this fraction was derived from N-terminal sequencing and MS data. The mass and the sequence of the major peptide in this product are summarised in Table 3. The data indicate that the active product is a sheep lactoperoxidase fragment.

**Table 3**

| Mass and amino acid sequence of the cationic antibacterial peptide obtained after hydrolysis on the cationic-exchange membrane using sheep cheese whey as starting material | | |
|---|---|---|
| Observed mass | Theoretical mass | Sequence |
| 1929.0 | 1929.2 | TQRKTRNGFRVPLARE |

### Example 4

### Preparation of a peptide mixture with antibacterial activity from bovine whey

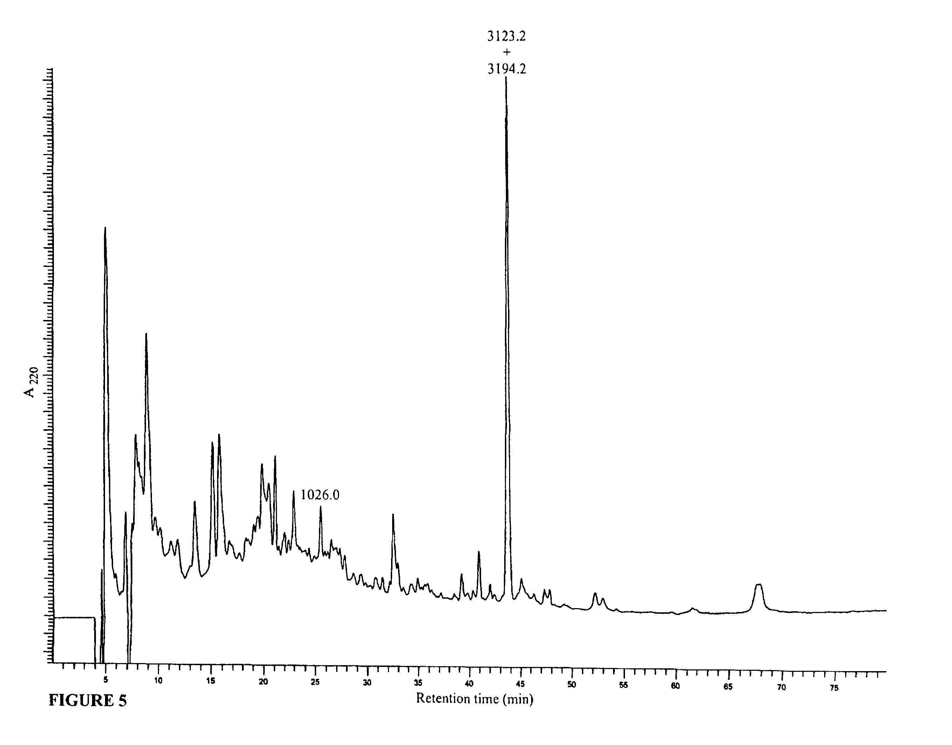

Experimental conditions were as described above for Example 2 with the exception that microfiltered bovine Gouda cheese whey (pH 6.5) was used as starting material. The active product obtained was monitored by RP-HPLC (Fig. 5) and MS, and the sequence of the major peptides in this fraction was derived from N-terminal sequencing and MS data. The masses and the sequences of the major peptides in this product are summarised in Table 4. The data indicate that these active products are fragments of bovine lactoferrin.

**Table 4**

| Masses and amino acid sequences of the cationic antibacterial peptides obtained after hydrolysis on the cationic-exchange membrane using bovine cheese whey as starting material | | |
|---|---|---|
| Observed mass | Theoretical mass | Sequence |
| 1026.0 | 1026.2 | APRKNVRW |
| 3123.2 | 3123.8 | FKC*RRWQWRMKKLGAPSITC*VRRAF |
| 3194.2 | 3194.9 | FKC*RRWQWRMKKLGAPSITC*VRRAFA |

| | | |
|---|---|---|
| * linked by a disulfide bridge | | |

### Example 5

### Preparation of a peptide mixture with antibacterial activity from purified bovine lactoferrin

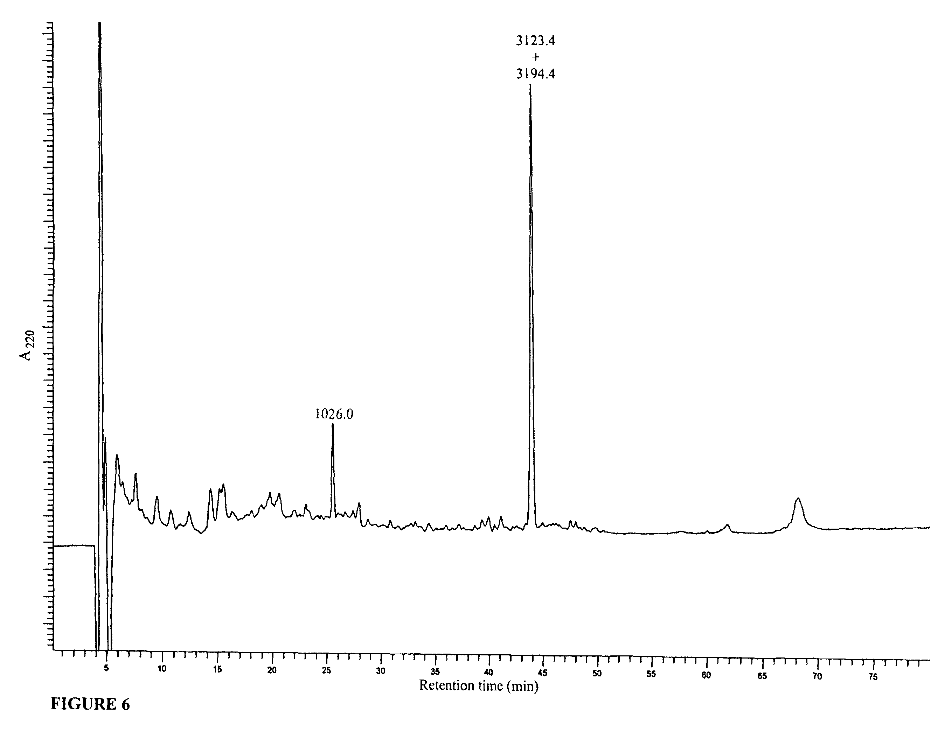

Experimental conditions were as described above for Example 2 with the exception that a solution of purified bovine lactoferrin in 10 mM sodium phosphate buffer (pH 7.0) was used as starting material. The active product obtained was monitored by RP-HPLC (Fig. 6) and MS, and the sequence of the major peptides in this fraction was derived from N-terminal sequencing and MS data. The masses and the sequences of the major peptides in this product are summarised in Table 5.

**Table 5**

| Masses and amino acid sequences of the cationic antibacterial peptides obtained after hydrolysis on the cationic-exchange membrane using a solution of purified lactoferrin as starting material. | | |
|---|---|---|
| Observed mass | Theoretical mass | Sequence |
| 1026.0 | 1026.2 | APRKNVRW |
| 3123.4 | 3123.8 | FKC*RRWQWRMKKLGAPSITC*VRRAF |
| 3194.4 | 3194.9 | FKC*RRWQWRMKKLGAPSITC*VRRAFA |

| | | |
|---|---|---|
| * linked by a disulfide bridge | | |

### Example 6

### Preparation of the lantibiotic nisin A from a cell extract containing nisin A precursor

For the production of nisin A precursor, *Lactococcus lactis* strain MG1614 harbouring the plasmid pNZ9111 [Van der Meer *et al*., J. Bacteriol. (1993) 175:2578-2588] was grown to the early stationary phase (final optical density at 600 nm 0.9-1.0, pH 6.2) in M17 broth (Difco Laboratories, Detroit, Ml, USA) supplemented with 0.5 w/v % glucose and 10 mg/ml erythromycin. The culture was centrifuged for 20 min at 2,500 × *g* to remove lactococcal cells. The supernatant was again centrifuged for 30 min at 10,000 × *g* and, after 10-fold dilution with bidistilled water, the pH was adjusted to 4.5-4.8 with acetic acid.

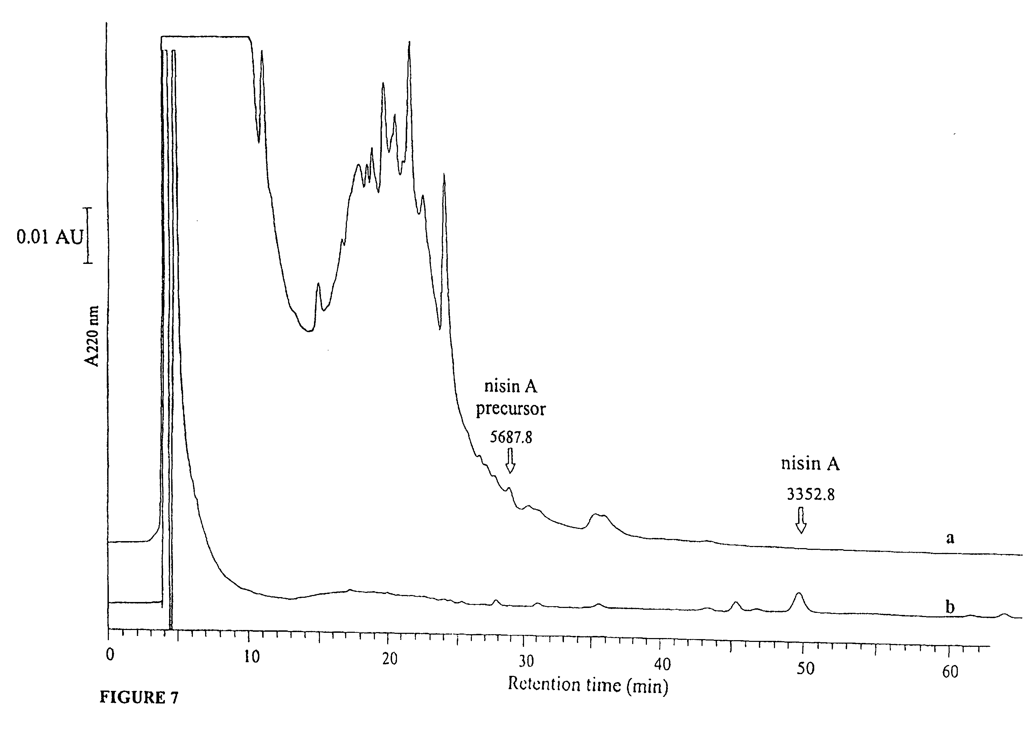

Prior to use, the cation-exchange membrane (Sartorius Sartobind S MA 100; Sartorius GmbH, Göttingen, Germany) was equilibrated with 10 mM ammonium acetate buffer, pH 4.5. The process was monitored by a UV detector with a 2-mm lightpath flow cuvette (Model EM-1 Econo UV Monitor, Bio-Rad Laboratories, Richmond, CA, USA). 3 L of the 10-fold diluted cell-free supernantant was pumped through the cation-exchange membrane at room temperature and at a flow rate of 20 ml/min which was generated by a peristaltic pump (Verder-Vleuten b.v., Vleuten, The Netherlands). The cation-exchange membrane was then washed with 10 mM sodium phosphate buffer, pH 7.0, to remove unbound material. The nisin precursor bound to the membrane was hydrolysed for 15 min at 37 °C by recycling, at 20 ml/min and at reverse flow, 50 ml of a solution of trypsin (Sigma Chemical Co., St. Louis, MO, USA) (0.1 mg/ml) in 10 mM sodium phosphate buffer, pH 7.0. After hydrolysis, the membrane was rinsed with 10 mM sodium phosphate buffer, pH 7.0, until baseline level was achieved. Finally, nisin was desorbed from the cation-exchange membrane with 1 M KCI in phosphate buffer, pH 7.0. This fraction showed activity against *Micrococcus flavus* when tested by a plate diffusion assay. The active product was monitored by reversed-phase high-performance liquid chromatography (RP-HPLC) (Fig. 7), and the isolated nisin A component was identified by mass spectrometry.

### Example 7

### Preparation of negatively charged peptides from β-casein

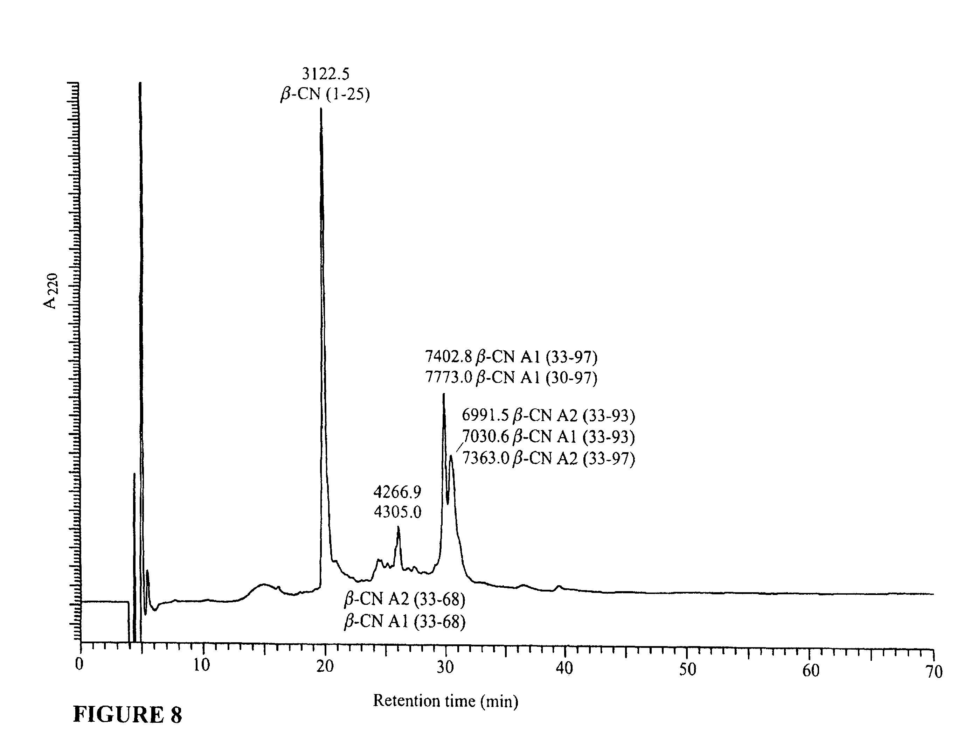

Prior to use, the anion-exchange membrane (Sartorius Sartobind Q MA 100; Sartorius GmbH, Göttingen, Germany) was equilibrated with 10 mM Tris-HCI buffer, pH 8.2. The process was monitored by a UV detector with a 2-mm lightpath flow cuvette (Model EM-1 Econo UV Monitor, Bio-Rad Laboratories, Richmond, CA, USA). 1 L of a solution of β-casein (0.1 mg/ml) in 10 mM Tris-HCI buffer, pH 8.2, was pumped through the anion-exchange membrane at room temperature and at a flow rate of 20 ml/min which was generated by a peristaltic pump (Verder-Vleuten BV, Vleuten, The Netherlands). The anion-exchange membrane was then washed with 10 mM sodium phosphate buffer, pH 7.0, to remove unbound material. The (3-casein bound to the membrane was hydrolysed for 14h at 37°C by recycling, at 20 ml/min and at reverse flow, 50 ml of a solution of trypsin (Sigma Chemical Co., St. Louis, MO, USA) (0.02 mg/ml) in 10 mM sodium phosphate buffer, pH 7.0. After hydrolysis and washing of the membrane with 10 mM Tris-HCI buffer, pH 8.2, the membrane-bound peptides were eluted with a linear gradient from 0 to 100% of 1 M NaCl in 10 mM Tris-HCI buffer, pH 8.2. The most strongly retained fraction was monitored by reversed-phase high-performance liquid chromatography (RP-HPLC) (Fig. 8), and the peptides in this fraction were identified by mass spectrometry.

### Example 8

### Preparation of positively charged peptides from egg-white

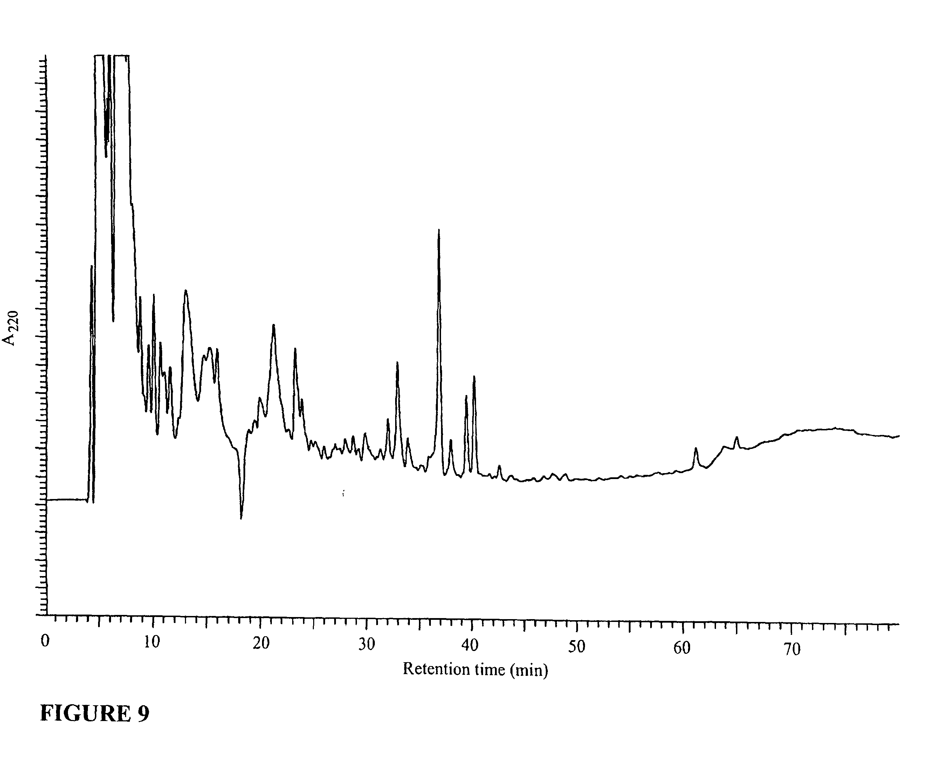

Prior to use, the cation-exchange membrane (Sartorius Sartobind S MA 100; Sartorius GmbH, Göttingen, Germany) was equilibrated with 20 mM HEPES buffer, pH 8.0. The process was monitored by a UV detector with a 2-mm lightpath flow cuvette (Model EM-1 Econo UV Monitor, Bio-Rad Laboratories, Richmond, CA, USA). The whites of three eggs were separated and diluted with 20 mM HEPES, pH 8.0, to give a 10% (w/v) suspension. The suspension was settled overnight at 4°C under stirring and the supernatant liquid was collected by centrifuging at 10000 x *g* for 30 min. Approx. 600 ml of this clear egg-white solution was pumped through the cation-exchange membrane at room temperature and at a flow rate of 20 ml/min which was generated by a peristaltic pump (Verder-Vleuten BV, Vleuten, The Netherlands). The cation-exchange membrane was then washed with 20 mM HEPES buffer, pH 8.0, to remove unbound material. The protein bound to the membrane was hydrolysed for 12 h at 60°C by recycling, at 20 ml/min and at reverse flow, 50 ml of a 1% (v/v) solution of Alcalase® (2.4 units/g; Novo Nordisk, Bagsvaerd, Denmark) in 20 mM HEPES buffer, pH 8.0. After hydrolysis and washing of the membrane with 20 mM HEPES buffer, pH 8.0, the membrane-bound peptides were eluted with a 7 M ammonia solution. This fraction was monitored by reversed-phase high-performance liquid chromatography (RP-HPLC). See Figure 9.

The present disclosure is therefore to be considered as in all respects illustrative and not restrictive, the scope of the invention being indicated by the appended Claims, and all changes which come within the meaning and range of equivalency are intended to be embraced therein.

## Claims

1. A process for the production of at least one peptide of interest from a biological fluid which comprises the steps of:
a) contacting said biological fluid comprising one or more proteins which contain the amino acid sequence of said peptide of interest with a chromatographic medium to adsorb said protein containing said sequence,
b) subjecting said adsorbed material to hydrolysis to fragment said protein and produce said peptide which remains substantially adsorbed,
c) washing the medium to remove unbound material,
d) desorbing said remained substantially adsorbed peptide from said chromatographic medium, and, optionally,
e) further purifying said desorbed peptide of interest.

2. The process according to claim 1, further comprising, before step b), the step of washing the medium to remove unbound material.

3. The process according to claim 1 or claim 2, wherein said chromatographic medium is a cation-exchanger.

4. The process according to claim 1 or claim 2, wherein said chromatographic medium is an anion-exchanger.

5. The process according to claim 1 or claim 2, wherein said chromatographic medium is a medium for hydrophobic interaction chromatography.

6. The process according to claim 1 or claim 2, wherein said chromatographic medium is a medium for affinity chromatography.

7. The process according to any one of claims 1 to 6, wherein said chromatographic medium comprises a chromatographic membrane.

8. The process according to any one of claims 1 to 7, wherein the hydrolysis is carried out enzymatically.

9. The process according to claim 8, wherein one or more enzymes are used selected from the group of pepsin, chymosin, trypsin, plasmin, chymotrypsin, *subtilisin*, and thermolysin.

10. The process according to any one of claims 1 to 9, wherein the biological fluid is selected from the group of milk, whey, blood, blood serum, egg white, culture cells, extracts from culture cells, and plant cells.

11. A peptide, obtainable by the method of any one of claims 1 to 10, having an amino acid sequence selected from the following sequences (1) - (8), or derivatives thereof having a primary amide at the carboxy end thereof, which derivatives do not interfere with any biological properties of the peptide:
(1) VYQHQKAMKPWIQPKT
(2) VYQHQKAMKPWIQPKTKVIPY
(3) VYQHQKAMKPWIQPKTKVIPYVRY
(4) VYQHQKAMKPWIQPKTKVIPYVRYL
(5) PEWSKC*YQWQRRMRKLGAPSITC*IRRTSA (* = linked by a disulfide bridge)
(6) PEWSKCYQWQRRMRKLGAPSITCIRRTSA
(7) TQRKTRNGFRVPLARE
(8) APRKNVRW.

12. Use of a peptide as claimed in claim 11, for the preparation of pharmaceutical compositions, preferably with antimicrobial and/or antiviral and/or antitumour activity.

## Patentansprüche

1. Verfahren zur Herstellung von mindestens einem Peptid von Interesse aus einer biologischen Flüssigkeit, welches die Schritte umfasst:
(a) Inkontaktbringen der biologischen Flüssigkeit, umfassend ein oder mehrere Protein(e), welche(s) die Aminosäuresequenz des Peptids von Interesse enthält/enthalten, mit einem Chromatographiemedium, um das Protein zu adsorbieren, welches die Sequenz enthält,
(b) Durchführen einer Hydrolyse mit dem adsorbierten Material, um das Protein zu fragmentieren und das Peptid herzustellen, das im Wesentlichen adsorbiert bleibt,
(c) Waschen des Mediums, um ungebundenes Material zu entfernen,
(d) Desorbieren des im Wesentlichen adsorbiert gebliebenen Peptids von dem Chromatographiemedium und, gegebenenfalls,
(e) zusätzlich Reinigen des desorbierten Peptids von Interesse.

2. Verfahren nach Anspruch 1, das zusätzlich vor Schritt (b) den Schritt des Waschens des Mediums umfasst, um ungebundenes Material zu entfernen.

3. Verfahren nach Anspruch 1 oder 2, wobei das Chromatographiemedium ein Kationenaustauscher ist.

4. Verfahren nach Anspruch 1 oder 2, wobei das Chromatographiemedium ein Anionenaustauscher ist.

5. Verfahren nach Anspruch 1 oder 2, wobei das Chromatographiemedium ein Medium für hydrophobe Wechselwirkungschromatographie ist.

6. Verfahren nach Anspruch 1 oder 2, wobei das Chromatographiemedium ein Medium für Affinitätschromatographie ist.

7. Verfahren nach einem der Ansprüche 1 bis 6, wobei das Chromatographiemedium eine Chromatographiemembran umfasst.

8. Verfahren nach einem der Ansprüche 1 bis 7, wobei die Hydrolyse enzymatisch ausgeführt wird.

9. Verfahren nach Anspruch 8, wobei ein oder mehrere Enzym(e) benutzt werden, ausgewählt aus der Gruppe von Pepsin, Chymosin, Trypsin, Plasmin, Chymotrypsin, Subtilisin und Thermolysin.

10. Verfahren nach einem der Ansprüche 1 bis 9, wobei die biologische Flüssigkeit ausgewählt ist aus der Gruppe von Milch, Molke, Blut, Blutserum, Eiweiß, Kulturzellen, Extrakten von Kulturzellen und Pflanzenzellen.

11. Peptid, erhältlich gemäß dem Verfahren nach einem der Ansprüche 1 bis 10, mit einer Aminosäuresequenz ausgewählt aus den folgenden Sequenzen (1) - (8) oder Derivaten davon mit einem primären Amid am Carboxyende davon, wobei die Derivate biologische Eigenschaften des Peptids nicht beeinträchtigen:
(1) VYQHQKAMKPWIQPKT
(2) VYQHQKAMKPWIQPKTKVIPY
(3) VYQHQKAMKPWIQPKTKVIPYVRY
(4) VYQHQKAMKPWIQPKTKVIPYVRYL
(5) PEWSKC*YQWQRRMRKLGAPSITC*IRRTSA (*=verbunden durch eine Disulfidbrücke)
(6) PEWSKCYQWQRRMRKLGAPSITCIRRTSA
(7) TQRKTRNGFRVPLARE
(8) APRKNVRW.

12. Verwendung eines Peptids nach Anspruch 11 zur Herstellung von Arzneimitteln, vorzugsweise mit antimikrobieller und/oder antiviraler und/oder Anti-Tumor-Aktivität.

## Revendications

1. Procédé de production d'au moins un peptide d'intérêt à partir d'un fluide biologique qui comprend les étapes de :
a) contact dudit fluide biologique comprenant une ou plusieurs protéines qui contiennent la séquence d'acides aminés dudit peptide d'intérêt avec un milieu chromatographique pour adsorber ladite protéine contenant ladite séquence,
b) soumission dudit matériau adsorbé à l'hydrolyse en fragments de ladite protéine et production dudit peptide qui reste essentiellement adsorbé,
c) lavage du milieu pour éliminer le matériau non lié,
d) désorption dudit peptide resté essentiellement adsorbé dudit milieu chromatographique, et, éventuellement,
e) autre purification dudit peptide d'intérêt désorbé.

2. Procédé selon la revendication 1, comprenant en outre, avant l'étape b), l'étape de lavage du milieu pour éliminer le matériau non lié.

3. Procédé selon la revendication 1 ou la revendication 2, dans lequel ledit milieu chromatographique est un échangeur de cations.

4. Procédé selon la revendication 1 ou la revendication 2, dans lequel ledit milieu chromatographique est un échangeur d'anions.

5. Procédé selon la revendication 1 ou la revendication 2, dans lequel ledit milieu chromatographique est un milieu pour chromatographie d'interaction hydrophobe.

6. Procédé selon la revendication 1 ou la revendication 2, dans lequel ledit milieu chromatographique est un milieu pour chromatographie d'affinité.

7. Procédé selon l'une quelconque des revendications 1 à 6, dans lequel ledit milieu chromatographique comprend une membrane chromatographique.

8. Procédé selon l'une quelconque des revendications 1 à 7, dans lequel l'hydrolyse est effectuée par voie enzymatique.

9. Procédé selon la revendication 8, dans lequel une ou plusieurs enzymes sont utilisées, choisies dans le groupe de la pepsine, la chymosine, la trypsine, la plasmine, la chymotrypsine, la subtilisine, et la thermolysine.

10. Procédé selon l'une quelconque des revendications 1 à 9, dans lequel le fluide biologique est choisi dans le groupe du lait, petit-lait, sang, sérum sanguin, blanc d'oeuf, des cellules en culture, des extraits de cellules en culture, et des cellules végétales.

11. Peptide, que l'on peut obtenir par le procédé selon l'une quelconque des revendications 1 à 10, ayant une séquence d'acides aminés choisie parmi les séquences suivantes (1) à (8), ou des dérivés de celles-ci ayant un amide primaire à leur extrémité carboxy, lesquels dérivés n'interfèrent avec aucune des propriétés biologiques du peptide :
(1) VYQHQKAMKPWIQPKT
(2) VYQHQKAMKPWIQPKTKVIPY
(3) VYQHQKAMKPWIQPKTKVIPYVRY
(4) VYQHQKAMKPWIQPKTKVIPYVRYL
(5) PEWSKC*YQWQRRMRKLGAPSITC*IRRTSA
(6) PEWSKCYQWQRRMRKLGAPSITCIRRTSA
(7) TQRKTRNGFRVPLARE
(8) APRKNVRW.
(* = lié par un pont disulfure)

12. Utilisation d'un peptide selon la revendication 11, pour la préparation de compositions pharmaceutiques, de préférence avec une activité antimicrobienne et/ou antivirale et/ou antitumorale.
